Europäisches Patentamt

(19) European Patent Office

Office européen des brevets

(11) Numéro de publication: **0 047 693**
**B1**

(12) **FASCICULE DE BREVET EUROPEEN**

(45) Date de publication du fascicule du brevet:
15.02.84

(21) Numéro de dépôt: 81401352.0

(22) Date de dépôt: 27.08.81

(51) Int. Cl.³: **C 07 C  59/90,** C 07 C  51/373,
C 07 C  51/41, C 07 C  51/487 //
C07C47/565, C07C47/575,
C07C47/58, C07C45/39

(54) Procédé de fabrication d'acides hydroxyarylglyoxyliques et de leurs sels alcalins et son application à la fabrication du para-hydroxyphénylglyoxylate de sodium.

(30) Priorité: 04.09.80  FR 8019103

(43) Date de publication de la demande:
17.03.82 Bulletin 82/11

(45) Mention de la délivrance du brevet:
15.02.84 Bulletin 84/7

(84) Etats contractants désignés:
BE CH DE FR GB LI

(56) Documents cités:
EP - A - 0 005 779
US - A - 2 062 205
US - A - 2 640 083

CHEMICAL ABSTRACTS, vol. 7, no. 9, 10 mai 1913, page 1509, Columbus, Ohio, US, F. MAUTHNER: "New synthesis of syringaic aldehyde"

(73) Titulaire: **SOCIETE FRANCAISE HOECHST Société anonyme dite:, 3, avenue du Général de Gaulle, F-92800 Puteaux (FR)**

(72) Inventeur: **Schouteeten, Alain, 17, rue de Normandie, F-95460 Ezanville (FR)**
Inventeur: **Christidis, Yani, 12 Rue de Constantinople, F-75008 Paris (FR)**
Inventeur: **Vallejos, Jean-Claude, 9, Rue Labat, F-75018 Paris (FR)**

(74) Mandataire: **Rinuy, Guy et al, 14, Avenue de la Grande Armée, F-75017 Paris (FR)**

## Procédé de fabrication d'acides hydroxyarylglyoxyliques et de leurs sels alcalins et son application à la fabrication du para-hydroxyphénylglyoxylate de sodium

La présente invention concerne un nouveau procédé de fabrication d'acides hydroxyarylglyoxyliques et de leurs sels alcalins et son application à la fabrication du para-hydroxyphenylglyoxylate de sodium cristallisé anhydre ou avec 2 molécules d'eau.

Les acides hydroxyarylglyoxyliques sont des matières premières précieuses pour accéder, entre autres, aux aldéhydes aromatiques hydroxylés par dégradation décarboxylante. La décarboxylation aisée des acides arylglyoxyliques et la fragilité des acides arylglycoliques à l'oxydation fournissant par dégradation décarboxylante l'aldéhyde aromatique correspondant, a rendu pendant très longtemps la préparation des acides arylglyoxyliques rédhibitoire par oxydation des acides arylglycoliques correspondants. Pour préparer ces acides on s'est adressé jusqu'à ces dernières années à des méthodes détournées soit par condensation du chlorure d'éthoxalyle sur un alcoxybenzène suivie d'une hydrolyse alcaline des fonctions ester et éther selon L. Bouveault; Bull. Soc. chim. France, 1898, (3), 19, 75, soit par hydrolyse en milieu aqueux d'un sulfate d'oxaloaryldiazonium obtenu par diazotation en milieu sulfurique d'un acide aminoarylglyoxylique issu généralement de l'oxydation permanganique en milieu aqueux alcalin d'une aminoacétophénone préalablement acylée à l'azote selon J. Aloy et Ch. Rabaut, Bull. Soc. chim. France, 1911, (4), 9, 763, soit par oxydation permanganique en milieu alcalin d'une hydroxyacétophénone selon le brevet italien n° 475 964, soit par hydrolyse alcaline d'un cyanure de benzoyle selon F. Francis et coll., Annalen, 1911 382, 206 soit par oxydation chromique d'un mandélamide selon M.S. Mashevkaya, khim., Prom., 1979, 2, 5–7 (C.A., 1979, 91, 211037j), soit enfin par réaction de Hoesch (K. Hoesch, Ber., 1915, 48, 1122).

Or, ces procédés de préparation soit utilisent des réactifs toxiques, dangereux ou onéreux, soit fournissent l'acide cherché avec un faible rendement, soit enfin nécessitent l'emploi de matières premières rares.

Récemment un procédé d'oxydation d'acides arylglycoliques en acides arylglyoxyliques a été décrit dans la demande de brevet européen N° 0.005.779. Selon ce procédé, l'oxydation est réalisée par l'oxygène en milieu aqueux alcalin en présence d'un catalyseur déposé sur noir de carbone et constitué par du platine ou du palladium activés par un sel de plomb ou de bismuth.

Mais ce procédé de préparation, exige des catalyseurs coûteux entraînant, du fait des pertes inéluctables lors des opérations de récupération et de recyclage, un coût de fonctionnement élevé.

Or, la Demanderesse a trouvé un nouveau procédé économique de fabrication d'acides hydroxyarylglyoxyliques à partir d'acides hydroxyarylglycoliques éliminant ces inconvénients. Ce procédé, objet de la présente invention, est un procédé d'oxydation catalytique par l'oxygène en milieu aqueux alcalin, caractérisé en ce qu'il est effectué à température ambiante et en catalyse homogène avec des ions cuivriques.

Les ions cuivriques sont connus comme étant susceptibles d'oxyder certaines structures carbonylées telles que par exemple les acyloïnes. Mais, jusqu'à présent, à la connaissance de la Demanderesse, lorsqu'ils avaient été utilisés pour oxyder des acides carboxyliques alpha hydroxylés ils provoquaient une scission oxydante en dérivé carbonylé présentant un atome de carbone de moins que l'acide de départ et en dioxyde de carbone.

C'est ainsi que, lors de l'étude de nouvelles synthèses de la vanilline, A. Guyot et A. Gry, Bull. Soc. chim. France, 1910, (4), 7, 902–913, ne purent oxyder l'acide hydroxy-4 méthoxy-3 phénylglycolique en acide hydroxy-4 méthoxy-3 phénylglyoxylique malgré l'emploi de conditions variées et en particulier l'utilisation du sulfate cuivrique; ils n'obtinrent que la vanilline ou des produits qui en dérivent par résinification.

Le procédé, objet de la présente invention, est réalisé à température ambiante ou à une température voisine de la température ambiante. Comme l'oxydation d'un alcool secondaire en carbonyle est exothermique, il est donc souvent nécessaire de refroidir le milieu réactionnel du cours de l'oxydation. En effet, la Demanderesse a constaté qu'une élévation de température était nuisible à la sélectivité de l'oxydation et l'orientait préférentiellement vers une dégradation oxydante décarboxylante en dérivé aldéhydique.

Le procédé, objet de la présente invention, est effectué en milieu aqueux alcalin, à pH supérieur ou égal à 11,5. Selon un mode avantageux, il est mis en œuvre au départ d'une solution aqueuse de concentration comprise entre 0,3 M et 2 M d'acide hydroxyarylglycolique et en présence d'un excès d'hydroxyde de métal alcalin, comme l'hydroxyde de sodium, par rapport à la stoechiométrie exigée pour salifier la fonction carboxylique et la ou les fonctions phénoliques présentes sur l'acide de départ. Avantageusement cet excès est supérieur ou égal à 0,5 mole d'hydroxyde de sodium par mole d'acide hydroxyarylglycolique mis en œuvre.

Le procédé, objet de la présente invention est exécuté en milieu aqueux alcalin, à pH supérieur ou égal à 11,5 et en présence d'ions cuivriques, $Cu^{++}$, en quantité catalytique. Avantageusement, on utilise de 0,1 à 0,01 atome gramme de cuivre sous forme d'ions cuivriques et préférentiellement de 0,05 à 0,08 atome gramme de cuivre sous forme d'ions cuivriques par mole d'acide hydroxyarylglycolique mis en œuvre. Les ions cuivriques sont avantageusement apportés dans le milieu réactionnel par addition d'un sel minéral ou organique de cuivre II, comme par exemple le sulfate cuivrique anhydre ou hydraté, l'acétate cuivrique.

Le procédé, objet de la présente invention, est mis en œuvre en présence d'oxygène ou d'un gaz en contenant. Etant donné la faible solubilité de l'oxygène dans l'eau à la pression et à la température ambiantes, le procédé est généralement exécuté sous une pression d'oxygène inférieure ou égale à 10 bars et avantageusement sous une pression d'oxygène de 8 bars. Lorsque le procédé est réalisé sous une pression d'oxygène plus faible, comme par exemple sous une pression de 2 bars au lieu de 8 bars, on observe une baisse de la vitesse réactionnelle nécessitant une durée réactionnelle légèrement plus longue pour obtenir le même rendement, mais la sélectivité du procédé n'est pas modifiée.

En fin de réaction, après filtration pour éliminer quelques impuretés mécaniques suivie, si nécessaire, d'une séparation à pH = 5 du dérivé aldéhydrique éventuellement formé, l'acide hydroxyarylglyoxylique obtenu est extrait du milieu réactionnel préalablement acidifié à pH = 0,5 avec un acide minéral fort tel que l'acide chlorhydrique concentré avec un solvant peu ou pas miscible à l'eau tel que l'acétate d'éthyle. Après élimination sous vide du solvant d'extraction, on isole l'acide hydroxyarylglyoxylique cherché, généralement sous forme cristallisée, suffisamment pur pour être utilisé tel quel, sinon, une simple recristallisation par chaud et froid dans un solvant convenable permet de le purifier. Une fois l'acide ainsi obtenu il est aisé, en utilisant des moyens connus en soi, d'obtenir par réaction de neutralisation, les sels correspondants.

La présente invention a aussi pour objet l'application de ce procédé à la préparation plus particulière du para-hydroxyphénylgyoxylate de sodium cristallisé pur anhydre ou solvaté avec deux molécules d'eau.

A la connaissance de la Demanderesse, ce sel anhydre ou hydraté n'est pas décrit dans la littérature et grâce à sa faible solubilité constatée dans l'eau froide, il peut constituer un moyen commode pour isoler et/ou purifier l'acide para-hydroxyphénylgyoxylique.

En effet, pour obtenir le para-hydroxyphénylglyoxylate de sodium cristallisé pur avec deux molécules d'eau de solvatation, il suffit, en fin de réaction d'oxydation de l'acide para-hydroxyphénylgycolique et après filtration, d'arrêter l'acidification du milieu réactionnel à pH = 5 avec de l'acide chlorhydrique concentré, puis de filtrer le précipité obtenu et ensuite de le sécher sous vide à poids constant à une température inférieure ou égale à 60°C. Le para-hydroxyphénylglyoxylate de sodium cristallisé pur anhydre est obtenu en séchant à 75°C sous vide, à poids constant, le para-hydroxyphénylglyoxylate de sodium cristallisé pur avec deux molécules d'eau de solvatation. L'acide correspondant peut alors être obtenu par déplacement de ce sel par un acide minéral fort selon des moyens connus en soi.

Les exemples suivants feront mieux comprendre l'invention.

Ils sont donnés uniquement à titre illustratif mais ne constituent pas des limitations de l'invention.

Exemple 1

On agite, sous une pression initiale d'oxygène de 8 bars et sous refroidissement extérieur de manière à maintenir la température interne entre 20 et 30°C, une solution obtenue en dissolvant dans 546 g d'eau:

141,2 g (3,53 moles) d'hydroxyde de sodium en pastilles,
186,16 g (1 mole) d'acide p-hydroxymandélique monohydraté,
11,2 g (0,07 mole) de sulfate cuivrique anhydre.

On peut suivre l'évolution de la réaction par chromatographie en phase liquide. Pour ce faire, on prélève périodiquement une prise d'essai dans le milieu réactionnel pour déterminer sa concentration en acides para-hydroxymandélique (PHM), para-hydroxyphénylglyoxylique (PHPG), para-hydroxybenzoïque (PHB) et en para-hydroxybenzaldéhyde (PHBZ). Ces déterminations sont effectuées par chromatographie liquide sur un appareil PHILIPS, à 25°C, sous une pression de 100 bars avec un débit de 1 cm³ par mn d'un mélange éluant eau-méthanol 80–20 acidifié et avec une détection UV à 240 nm. Les résultats obtenus exprimés en moles (±0,05 mole) en fonction du temps exprimé en minutes sont rapportés dans le tableau I.

Tableau 1

| Temps | PHM | PHPG | PHB | PHBZ | Total |
|-------|------|------|-----|------|-------|
| 0 | 1 | 0 | 0 | 0 | 1 |
| 30 | 0,89 | 0,06 | 0 | 0 | 0,95 |
| 60 | 0,88 | 0,11 | 0 | 0 | 0,99 |
| 90 | 0,79 | 0,18 | 0 | 0,02 | 0,99 |
| 120 | 0,72 | 0,28 | 0 | 0,02 | 1,02 |
| 240 | 0,35 | 0,69 | 0 | 0,02 | 1,06 |
| 300 | 0,26 | 0,75 | 0 | 0,05 | 1,06 |
| 1320 | 0,02 | 1 | 0 | 0,05 | 1,07 |

Après 22 heures d'agitation à 25–30°C et sous une pression d'oxygène de 8 bars, le milieu réactionnel ramené à la pression ambiante est filtré pour éliminer les impuretés mécaniques, puis le filtrat maintenu à température ambiante est acidifié à pH = 5 avec de l'acide chlorhydrique concentré. Le sel de sodium de l'acide p-hydroxyphénylglyoxylique cristallise, on l'essore, on le lave ensuite par empâtage avec 100 cm³ d'acétate d'éthyle puis on le sèche sous vide à 60°C à poids constant. On recueille ainsi 202,15 g (0,9 mole) de produit cristallisé avec deux molécules d'eau soit un rendement de 90% de la théorie calculée par rapport à l'acide de départ.

Ce sel peut être recristallisé par chaud et froid dans 4,5 volumes d'eau avec un rendement de 83%. Séché à 60°C sous vide il conserve son eau de cristallisation, par contre séché à 75°C sous vide durant 2 heures, il conduit au produit anhydre. Il présente à 20°C une solubilité dans l'eau de 2,5 g pour 100 g.

## Microanalyses

Sur produit séché à 60°C à poids constant $C_8H_5O_4Na,_2H_2O$

|  | C(%) | H(%) | $H_2O$(%)* |
|---|---|---|---|
| Calculés: | 42,86 | 4,05 | 16,07 |
| Trouvés: | 43,0 | 4,2 | 16,3 |

*déterminés selon la méthode de K. Fischer.

Sur produit séché à 75°C à poids constant $C_8H_5O_4Na$

|  | C(%) | H(%) | $H_2O$(%) |
|---|---|---|---|
| Calculés: | 51,07 | 2,68 | 0 |
| Trouvés: | 51,2 | 2,7 | 0 |

A la connaissance de la Demanderesse ce produit n'est pas décrit dans la littérature.

Ce sel de sodium mis en suspension dans de l'eau acidifiée à pH = 0,5 par l'acide chlorhydrique concentré conduit quantitativement à l'acide p-hydroxyphénylglyoxylique F = 177–178°C (R.D. Sprenger et collaborateurs, J. Amer., Chem. Soc., 1950, 72, 2874, F = 177,5–178°C).

## Exemples 2–9

Le tableau II illustre les exemples 2 à 9. Dans ce tableau, les réactifs sont exprimés en moles, les pressions d'oxygène en bars, les températures en °C, les durées réactionnelles en heures et les concentrations en grammes d'acide para-hydroxymandélique pour 100 g de solution réactionnelle. Les rendements en acide para-hydroxyphénylglyoxylique ont été déterminés sur les milieux bruts réactionnels par chromatographie liquide; ils sont exprimés en pourcentages des théories calculées par rapport à l'acide para-hydroxymandélique mis en œuvre.

La sélectivité exprimée en pourcentages est le rapport molaire de l'acide para-hydroxyphénylglyoxylique sur la somme acide para-hydroxybenzoïque acide para-hydroxyphénylglyoxylique et para-hydroxybenzaldéhyde formés durant la réaction. Cette sélectivité est donné à ±1%.

Le mode opératoire utilisé est similaire à celui décrit dans l'exemple 1.

## Tableau 2

| réactifs conditions et résultats | n° des exemples | | | | | | | |
|---|---|---|---|---|---|---|---|---|
|  | 2 | 3 | 4 | 5 | 6 | 7 | 8 | 9 |
| PHM | 1 | 1 | 1 | 1 | 1 | 1 | 1 | 1 |
| Hydroxyde de sodium | 5,53 | 5,53 | 5,53 | 5,53 | 2 | 2,53 | 2,53 | 2,53 |
| Sulfate cuivrique anhydre | 0,072 | 0,072 | 0,071 | 0 | 0,071 | 0,071 | 0,071 | 0,071 |
| eau | 51,2 | 51,2 | 26,85 | 23,4 | 36,6 | 18,93 | 33,53 | 33,53 |
| Poids totaux | 1322,3 | 1322,3 | 883,9 | 821,9 | 918,3 | 621,5 | 884,6 | 884,6 |
| Concentrations | 12,7 | 12,7 | 19 | 20,45 | 18,3 | 27,0 | 19 | 19 |
| Pressions | 8 | 8 | 8 | 8 | 8 | 8 | 2 | 8 |
| Températures | 50 | 25 → 43 | 27±2 | 27±2 | 27±2 | 20±2 | 19 → 30 | 23 → 31 |
| Durées | 11 | 22 | 20 | 22 | 21 | 22 | 21 | 7 |
| Rendements | 86 | 90 | 92,8 | 11 | 22 | 94,5 | 74 | 85,6 |
| Sélectivités | 90 | 95 | 99,8 |  |  | 96,7 | 96,5 | 96,7 |

## Exemple 10

On agite durant 30 heures sous une pression initiale de 8 bars d'oxygène et sous refroidissement extérieur de manière à maintenir la température du milieu réactionnel entre 20 et 30°C, une solution obtenue en dissolvant dans 258 g (14,3 moles) d'eau:

90,6 g (2,26 moles) d'hydroxyde de sodium en pastilles;
101,25 g (0,5 mole) d'acide chloro-3 hydroxy-4-mandélique;
9 g (0,036 mole) de sulfate cuivrique pentahydraté.

En fin de réaction, le milieu réactionnel ramené à la pression ambiante est filtré, puis le filtrat maintenu à température ambiante, est acidifié à pH = 0,5 avec de l'acide chlorhydrique concentré et il est ensuite soumis à des extractions successives avec de l'acétate d'éthyle. On isole ainsi après élimination du solvant d'extraction 87,4 g (0,436 mole) d'acide chloro-3 hydroxy-4 phénylglyoxylique présentant un point de fusion de 116–117°C (F = 114–116°C, FUSISAWA PHARMACEUTICAL Comp. Ltd FR 2299869) soit un rendement de 87,2% de la théorie.

Exemple 11

On opère selon l'exemple 10, au départ d'une solution de:

90,6 g (2,26 moles) d'hydroxyde de sodium en pastilles
123,1 g (0,5 mole) d'acide diméthoxy-3,5-hydroxy-4-mandélique monohydraté,
9,0 g (0,036 mole) de sulfate cuivrique pentahydraté
308 g (17,1 moles) d'eau

En fin de réaction, le milieu réactionnel ramené à la pression ambiante est filtré puis le filtrat maintenu à température ambiante est acidifié à pH = 5 avec de l'acide chlorhydrique concentré et il est ensuite soumis à des extractions successives avec de l'acétate d'éthyle. On isole ainsi après élimination sous vide du solvant d'extraction, 40,7 g (0,223 mole) de diméthoxy-3,5 hydroxy-4 benzaldéhyde F = 113°C (F = 113°C Beil., 8, 391). Les eaux mères d'extractions réunies sont ensuite acidifiées à pH = 0,5 avec de l'acide chlorhydrique concentré puis elles sont soumises à des extractions répétées avec de l'acétate d'éthyle.

On isole ainsi après élimination sous vide du solvant d'extraction 21,9 g (0,096 mole) d'acide diméthoxy-3,5 hydroxy-4-phénylglyoxylique présentant un point de fusion de 134±1°C (F: 128°C Mauthner, Annalen, 1913, 395, 276) soit un rendement de 19,2% de la théorie.

Exemple 12

On opère selon l'exemple 11 a départ d'une solution de:

99 g (0,5 mole) d'acide hydroxy-4-méthoxy-3 mandélique;
90,6 g (2,26 mole) d'hydroxyde de sodium en pastilles;
9 g (0,036 mole) de sulfate cuivrique pentahydraté;
258 g (14,3 moles) d'eau

On isole après traitements, 30,7 g (0,2 mole) de hydroxy-4 méthoxy-3 benzaldéhyde. F = 81–83°C (F = 81–83°C Beil., 8, 247) et 41,7 g (0,213 mole) d'acide hydroxy-4 méthoxy-3 phénylglyoxylique, F = 133–134°C (F = 133–134°C, Beil., 10, 988) soit un rendement de 42,6% de la théorie.

Il va de soi que la présente invention n'a été décrite qu'à titre purement explicatif et nullement limitatif et que toute modification utile pourra y être apportée sans sortir de son cadre tel que défini par les revendications ci-après.

**Revendications**

1. Procédé de fabrication d'acides hydroxyarylglyoxyliques à partir d'acides hydroxyarylglycoliques par oxydation catalytique en milieu aqueux alcalin, à pH supérieur ou égal à 11,5 par l'oxygène ou par gaz en contenant, caractérisé en ce qu'il est effectué en phase homogène à une température inférieure ou égale à 30°C et en présence d'ions cuivriques et qu'en fin de réaction l'acide cherché est isolé du milieu réactionnel par acidification à pH environ 0,5.

2. Procédé selon la revendication 1, caractérisé par le fait que l'on utilise de 0,1 à 0,01 atome gramme de cuivre sous forme d'ions cuivriques par mole d'acide hydroxyarylglycolique mis en œuvre.

3. Procédé selon la revendication 1 ou 2, caractérisé par le fait que les ions cuivriques sont apportés dans le milieu réactionnel par un sel minéral ou organique de cuivre II.

4. Procédé selon la revendication 3, caractérisé par le fait que le sel minéral ou organique de cuivre II est le sulfate cuivrique anhydre ou hydraté ou l'acétate cuivrique.

5. Procédé selon l'une quelconque des revendications 1 à 4, caractérisé par le fait qu'il est effectué au départ d'une solution aqueuse de concentration comprise entre 0,3 et 2 M d'acide hydroxyarylglycolique et en présence d'un excès d'hydroxyde alcalin comme l'hydroxyde de sodium par rapport à la stœchiométrie exigée pour salifier la fonction carboxylique et la ou les fonctions phénoliques présentes sur l'acide de départ.

6. Application du procédé selon l'une quelconque des revendications 1 à 5 à la fabrication du para-hydroxyphénylglyoxylate de sodium, caractérisé en ce que l'acide de départ est l'acide para-hydroxymandélique et qu'en fin de réaction ledit sel est isolé du milieu réactionnel par acidification à pH = 5.

7. Application selon la revendication 6, caractérisée en ce que le para-hydroxyphénylglyoxylate de sodium isolé est à son tour acidifié à pH environ 0,5 pour donner l'acide para-hydroxyphénylglyoxylique.

8. Application selon la revendication 6, caractérisée en ce que le sel de sodium isolé est séché sous vide à poids constant soit à 60°C pour donner le sel correspondant cristallisé avec 2 molécules d'eau soit à 75°C pour donner le sel correspondant anhydre.

**Patentansprüche**

1. Verfahren zur Herstellung von Hydroxyarylglyoxylsäuren aus Hydroxyarylglykolsäuren durch katalytische Oxidation in wässrigem alkalischem Medium bei einem pH-Wert gleich oder oberhalb von 11,5 durch Sauerstoff oder sauerstoffhaltiges Gas, dadurch gekennzeichnet, dass es in homogener Phase bei einer Temperatur gleich oder unterhalb von 30°C und in Anwesenheit von Kupfer(II)-ionen durchgeführt wird, und dass die gewünschte Säure am Schluss der Reaktion aus dem Reaktionsmedium durch Ansäuren auf einen pH-Wert von ungefähr 0,5 isoliert wird.

2. Verfahren nach Anspruch 1, dadurch gekennzeichnet, dass 0,1 bis 0.01 Atomgramm Kupfer in Form von Kupfer(II)-ionen pro Mol eingesetzter Hydroxyarylglykolsäure verwendet werden.

3. Verfahren nach Anspruch 1 oder 2, dadurch gekennzeichnet, dass die Kupfer(II)-ionen in das Reaktionsmedium in Form eines anorganischen oder organischen Kupfer(II)-salzes eingebracht werden.

4. Verfahren nach Anspruch 3, dadurch gekennzeichnet, dass als anorganisches oder organisches Kupfer(II)-salz wasserfreies oder hydratisiertes Kupfer(II)-sulfat oder Kupfer(II)-acetat verwendet wird.

5. Verfahren nach einem der Ansprüche 1 bis 4, dadurch gekennzeichnet, dass es beim Start mit einer wässrigen Lösung mit einer Konzentration zwischen 0,3 und 2 M Hydroxyarylglykolsäure und in Anwesenheit eines Überschusses eines Alkalihydroxids, wie Natriumhydroxid, bezogen auf die stöchiometrischen Erfordernisse zur Salzbildung mit der Carboxylgruppe und der oder den in der Ausgangssäure vorhandenen phenolischen Gruppe/n durchgeführt wird.

6. Anwendung des Verfahrens nach einem der Ansprüche 1 bis 5 zur Herstellung von Natriumpara-hydroxyphenylglyoxylat, dadurch gekennzeichnet, dass als Ausgangssäure para-Hydroxymandelsäure verwendet wird, und dass am Ende der Reaktion das Salz aus dem Reaktionsmedium durch Ansäuren auf einen pH-Wert von 5 isoliert wird.

7. Anwendung gemäss Anspruch 6, dadurch gekennzeichnet, dass das isolierte Natrium-parahydroxyphenylglyoxylat seinerseits auf einen pH-Wert von ungefähr 0,5 zur Gewinnung von para-Hydroxyphenylglyoxylsäure angesäuert wird.

8. Anwendung gemäss Anspruch 6, dadurch gekennzeichnet, dass das isolierte Natriumsalz unter Vakuum bis zur Gewichtskonstanz entweder bei 60°C zur Gewinnung des entsprechenden, mit 2 Molekülen Wasser kristallisierten Salzes oder bei 75°C zur Gewinnung des entsprechenden, wasserfreien Salzes getrocknet wird.

Claims

1. A process for preparation of hydroxyaryl-glyoxylic acids from hydroxyarylglycolic acids through catalytic oxidation in an aqueous alkaline medium to a pH higher than, or equal to, 11.5 by oxygen or an oxygen-containing gas, characterized in that it is carried out in a homogeneous phase at a temperature lower than, or equal to, 30°C, and in the presence of cupric ions, and that the end of the reaction, the desired acid is isolated by acidification from the reactional medium.

2. A process according to claim 1, characterized by using from 0,1 to 0,01 atom gram of copper in form of cupric ions per mole of hydroxyarylglycolic acid used.

3. A process according to claim 1 or claim 2, characterized by bringing the cupric ions into the reactional medium by a mineral or organic salt of copper II.

4. A process according to claim 3, characterized in that the mineral or organic salt of copper II is the anhydrous or hydrated cupric sulfate or the cupric acetate.

5. A process according to any one of claims 1 to 4, characterized in that it is carried out starting from an aqueous solution of concentration comprised between 0.3 and 2M of hydroxyarylglycolic acid and in the presence of an excess of an alkaline hydroxide such as sodium hydroxide with respect to the required stoichiometry for salifying the carboxylic function and the phenolic function(s) present on the starting acid.

6. An application of the process according to any one of claims 1 to 5 for the preparation of sodium parahydroxyphenylglyoxylate, characterized in that the starting acid is para-hydroxymandelic acid and at the end of the reaction said salt is isolated from the reactional medium by acidification to pH = 5.

7. An application according to claim 6, characterized in that the isolated sodium para-hydroxyphenylglyoxylate is acidified to pH of about 0.5 to supply para-hydroxyphenylglyoxylic acid.

8. An application according to claim 6, characterized in that the isolated sodium salt is dried under vacuum at constant weight either at 60°C to produce the corresponding salt crystallized with 2 molecules of water, or at 75°C to produce the corresponding anhydrous salt.